# EUROPEAN PATENT APPLICATION

(11) **EP 3 338 747 A1**
(43) Date of publication of application: **27.06.2018**
(21) Application number: 17206807.4
(22) Date of filing: 12.12.2017
(51) Int. Cl.: A61F 13/494, A61F 13/496

(54) **PULL-ON DISPOSABLE DIAPER**

(30) Priority: 21.12.2016 JP 2016248178
(71) Applicant: Unicharm Corporation, Ehime 799-0111 (JP)
(72) Inventor: Mukai, Hirotomo, Kanonji-shi, Kagawa 769-1602 (JP)
(74) Representative: Dolleymores

(57) **Abstract**

An object of the present invention is to improve fitting performance of a front waist section in a pull-on disposable diaper.

There is provided a pull-on disposable diaper (1) having a vertical direction, a lateral direction and a thickness direction that are orthogonal to one another, the pull-on disposable diaper (1) including: an absorbent main body (10) that absorbs liquid; a front waist section (3) positioned at one end part of the absorbent main body (10) in a longitudinal direction; and a back waist section (4) positioned at another end part of the absorbent main body (10) in the longitudinal direction, the front waist section (3) having a lower end part on which an elastic member (361g) that is extendable in the lateral direction is provided, the elastic member (361g) including an inclined section (361gc) that is inclined obliquely upward from an outside toward an inside in the lateral direction, at least in a region where the elastic member (361g) overlaps with the absorbent main body (10) in the thickness direction, the elastic member (361g) being discontinuous at a center part of the absorbent main body (10) in the lateral direction.

## Description

### TECHNICAL FIELD

The present invention relates to pull-on disposable diapers.

### BACKGROUND ART

Conventionally, as an absorbent article such as a diaper, a pull-on disposable diaper is known that is constituted of an absorbent main body that absorbs excrement and the like, and waist belt sections (waist sections) positioned at both end parts of this absorbent main body in a longitudinal direction. In such a disposable diaper (diaper), elastic members such as elastic strings are provided at the front and back waist sections . Stretch of these elastic members acts to increase fitting performance of the waist in wearing the diaper. For example, PTL 1 discloses a diaper where elastic members that are continuous in a lateral direction are provided so as to have a part overlapping an absorbent main body, at a lower side (that is, a leg side) end part of the front waist section in a vertical direction.

### Citation List

### Patent Literature

PTL 1: WO2016/99362

### SUMMARY OF INVENTION

### Technical Problem

With the diaper in PTL 1, stretching force by the elastic members can act entirely along the waist of a wearer. However, at the part where the elastic members overlap the absorbent main body in the front waist belt section (waist section), force that contracts the absorbent main body in the lateral direction acts. Thus, the fitting performance is possibly degraded, and side leakage of the excrement possibly occurs. In the diaper in PTL 1, since the stretch by the elastic members acts only in the lateral direction of the front waist section, when the wearer of the diaper moves his/her own body (for example, when taking a forward-inclined posture), the front waist section contracts in the vertical direction, and riding-up occurs in some cases. Thus, it is difficult to ensure satisfactory fitting performance.

The present invention has been made in consideration of the conventional problems as described above. An object of the present invention is to improve fitting performance of a front waist section in a pull-on disposable diaper.

### Solution to Problem

A primal invention to achieve the above-described object is a pull-on disposable diaper having a vertical direction, a lateral direction and a thickness direction that are orthogonal to one another, the pull-on disposable diaper including: an absorbent main body that absorbs liquid; a front waist section positioned at one end part of the absorbent main body in a longitudinal direction; and a back waist section positioned at another end part of the absorbent main body in the longitudinal direction, the front waist section having a lower end part on which an elastic member that is extendable in the lateral direction is provided, the elastic member including an inclined section that is inclined obliquely upward from an outside toward an inside in the lateral direction, at least in a region where the elastic member overlaps with the absorbent main body in the thickness direction, the elastic member being discontinuous at a center part of the absorbent main body in the lateral direction.

Other features of the present invention will be made clear by the description and attached drawings.

### Advantageous Effects of Invention

With the present invention, the fitting performance of the front waist section can be improved in the pull-on disposable diaper.

### BRIEF DESCRIPTION OF DRAWINGS

Fig. 1 is a plan view of a diaper 1 in a developed and stretched state.
Fig. 2 is a schematic perspective view when the diaper 1 in a pull-on state is viewed from a front.
Fig. 3A is a schematic cross-sectional view taken along a line A-A in Fig. 1.
Fig. 3B is a schematic cross-sectional view taken along a line B-B in Fig. 1.
Fig. 4 is an explanatory view of disposition of dotted compressed portions 15.
Fig. 5 is an enlarged plan view illustrating a front waist section 3 in the developed and stretched state.
Fig. 6 is an explanatory view of action of force by an elastic member 361g in wearing the diaper 1.
Fig. 7 is an explanatory view of a modification of the disposition of the dotted compressed portions 15.

### DESCRIPTION OF EMBODIMENTS

At least the following matters will become clear through the description of the present specification and the accompanying drawings.

Disclosed is a pull-on disposable diaper having a vertical direction, a lateral direction and a thickness direction that are orthogonal to one another, the pull-on disposable diaper including: an absorbent main body that absorbs liquid; a front waist section positioned at one end part of the absorbent main body in a longitudinal direction; and a back waist section positioned at another end part of the absorbent main body in the longitudinal direction, the front waist section having a lower end part on which an elastic member that is extendable in the lateral direction is provided, the elastic member including an inclined section that is inclined obliquely upward from an outside toward an inside in the lateral direction, at least in a region where the elastic member overlaps with the absorbent main body in the thickness direction, the elastic member being discontinuous at a center part of the absorbent main body in the lateral direction.

With such a pull-on disposable diaper, force that widens the front waist section in the vertical direction acts in wearing due to stretching force of the inclined section of the elastic member. Thus, bending and riding-up are less likely to occur. This can improve fitting performance of the front waist section. The front waist section is fixedly fitted, and thus the absorbent main body can be more strongly supported. This can restrain the absorbent main body from drooping down even when this absorbent main body absorbs the excrement and increases its weight.

In this pull-on disposable diaper, it is preferable that the absorbent main body includes a liquid-absorbent absorbent core, and the elastic member includes a part overlapping with the absorbent core in the thickness direction.

With such a pull-on disposable diaper, the absorbent core can be widened in the lateral direction and the vertical direction as well as the absorbent main body. This can allow the absorbent core to be likely to fit the body of the wearer to efficiently restrain leakage or the like of the excrement.

In this pull-on disposable diaper, it is preferable that the absorbent core has a surface on which a plurality of dotted compressed portions are provided, and the plurality of dotted compressed portions are disposed at an equal pitch in the vertical direction.

With such a pull-on disposable diaper, base points for folding are likely to be equally formed in the longitudinal direction of the absorbent core. That is, in wearing the diaper, the absorbent core can be likely to deform uniformly in the longitudinal direction. This allows the absorbent core to be likely to effortlessly fit along a roundness of the body. Further, the stretching force of the elastic member widens the absorbent core. Thus, even when a large number of base points for folding are formed, extra creases or the like are less likely to be formed, thereby allowing the absorbent core to be likely to fit the body of the wearer.

In this pull-on disposable diaper, it is preferable that the absorbent core has a surface on which a plurality of dotted compressed portions are provided, and the plurality of dotted compressed portions are disposed with a first pitch and a second pitch different in length from the first pitch in the lateral direction.

With such a pull-on disposable diaper, a part where the pitch of the dotted compressed portions is narrow and the base points for folding are densely formed, and a part where the pitch of the dotted compressed portions is wide and the base points for folding are sparsely formed are mixed in the lateral direction of the absorbent core. Accordingly, the absorbent core can be deformed concentratedly and can be made less likely to deform from a planar shape. Thus, this can allow the absorbent core to be likely to freely deform in the lateral direction while maintaining the plane in accordance with a body shape of the wearer. In this way, the absorbent core becomes likely to further fit the body.

In this pull-on disposable diaper, it is preferable that the absorbent core has a surface on which a plurality of dotted compressed portions are provided, and the absorbent core includes a part where a number of the dotted compressed portions disposed per unit area is large, and a part where a number of the dotted compressed portions disposed per unit area is small.

With such a pull-on disposable diaper, the base points for folding are to be formed more easily on the part where a disposition density of the dotted compressed portions is high than the part where a disposition density is low. Having a region that is likely to deform and a region that is less likely to deform on an identical plane restrains the absorbent core from getting deformation habit in a specific direction. That is, the deformation of the absorbent core becomes irregular to be likely to follow a complicated curved surface of the body. This allows the surface fitting of the absorbent core with respect to the body of the wearer to be likely achieved, in wearing the diaper.

In this pull-on disposable diaper, it is preferable that the front waist section includes a waist elastic member that is extendable in the lateral direction, in an upper region in the vertical direction with respect to a region where the elastic member is disposed, at least a part of the waist elastic member is discontinuous at a center part in the lateral direction, and a position of an inner end of the elastic member and a position of an inner end of the waist elastic member are different in the lateral direction.

With such a pull-on disposable diaper, a range on which contractile force by the waist elastic member acts and a range on which contractile force by the elastic member acts can be adjusted in the lateral direction. This can change a point of action and a magnitude of force when the front waist section is pulled downward, thus improving fitting feeling of a target wearer.

In this pull-on disposable diaper, it is preferable that the front waist section includes: an inclined-section adhesion region to which the inclined section is joined; and a waist-elastic-member adhesion region to which the waist elastic member is joined without overlapping the inclined-section adhesion region.

With such a pull-on disposable diaper, the force by the front waist elastic member and the force by the inclined section of the elastic member become less likely to interfere with one another. Accordingly, this is likely to restrain the loss of assumed widening effect and the local tight fastening caused by a change in direction where the absorbent main body and the front waist section are pulled.

In this pull-on disposable diaper, it is preferable that the absorbent main body includes a liquid-absorbent absorbent core, and the waist elastic member does not have a part overlapping with the absorbent core in the thickness direction.

Such a pull-on disposable diaper can restrain a contraction amount in the lateral direction of the waist elastic member from decreasing at an overlapping part caused by overlapping with the absorbent core whose rigidity is high. Accordingly, the waist section becomes likely to contract over a wide range in the lateral direction to improve the fitting performance of the waist section in wearing the diaper.

In this pull-on disposable diaper, it is preferable that a right and left pair of the elastic members are provided along the lateral direction, and the inclined section has inclination angles with respect to the lateral direction, which are different at right and left.

With such a pull-on disposable diaper, the elastic members (the inclined sections) asymmetrically disposed can adjust a magnitude and a direction of the force that acts on the front waist section. This facilitates achievement of optimum fitting performance in wearing the diaper corresponding to the condition of the target wearer. For example, when the target wearer is a person whose one hand is paralyzed, an operation that raises the waist section at one side in the lateral direction is facilitated, and the fitting performance of the waist section is improved at the other side in the lateral direction.

In this pull-on disposable diaper, it is preferable that a right and left pair of the elastic members are provided along the lateral direction, the elastic members each include a straight section disposed along the lateral direction, and a curving section curved at a certain curvature between the inclined section and the straight section, and curvatures of the curving sections are different at right and left.

With such a pull-on disposable diaper, the stretching force by the straight section and the stretching force by the inclined section are likely to smoothly coordinate. Then, since the curvatures of the curving sections are different at right and left, it becomes possible to adjust the magnitude and the direction of the force that acts on the waist section. This facilities the achievement of the optimum fitting performance in wearing the diaper in accordance with the condition of the target wearer (for example, the person whose one hand is paralyzed).

### === Embodiment ===

### Basic Configuration of Diaper 1

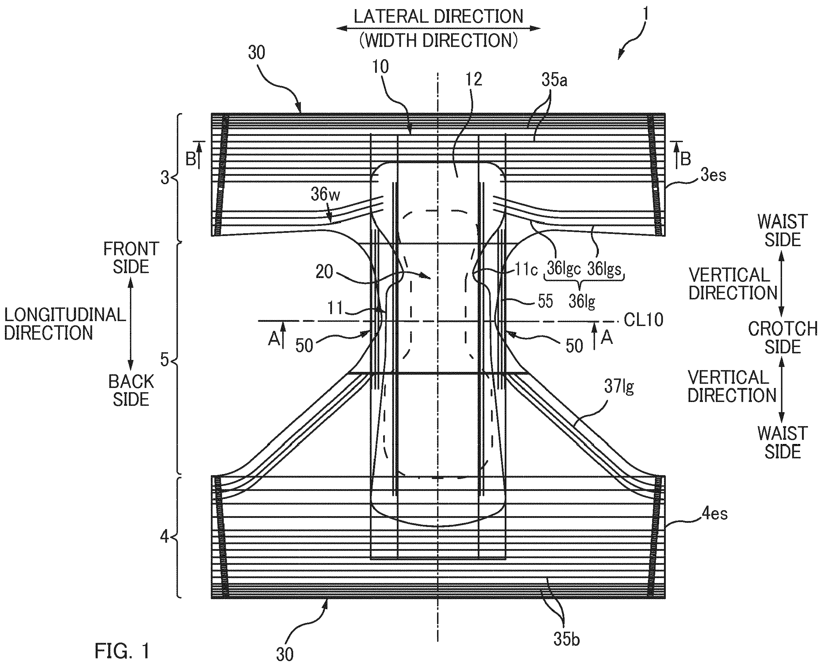

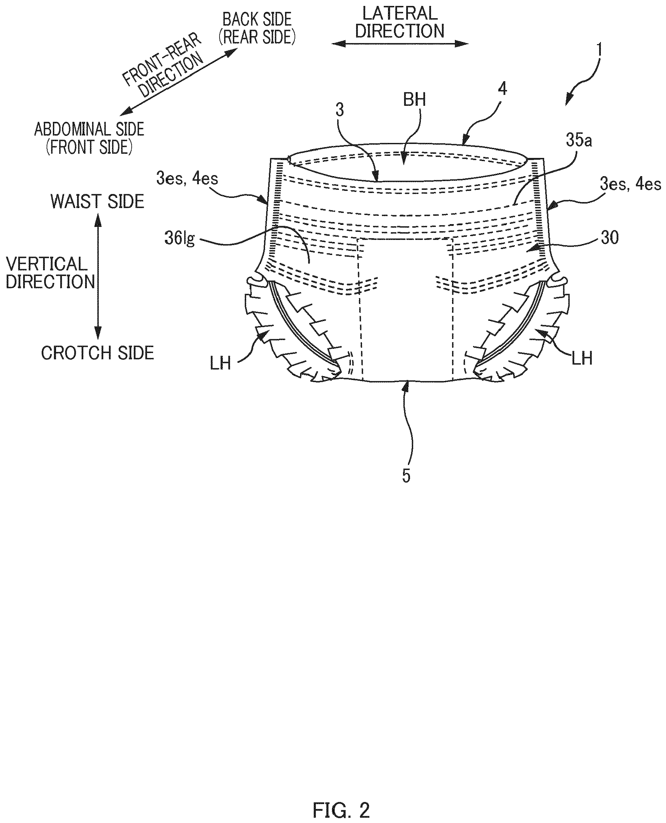

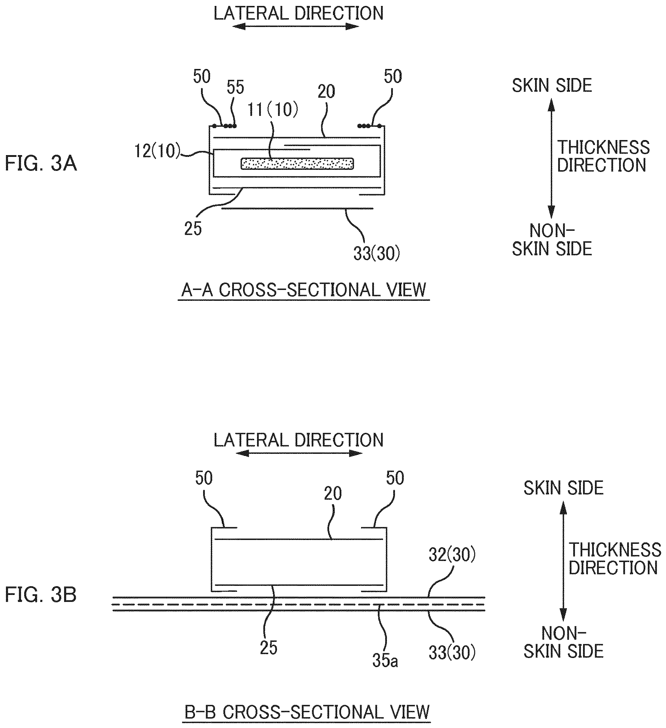

As one example of the absorbent article dealt in this embodiment, the following describes a basic configuration of a pull-on disposable diaper 1 (hereinafter also referred to as a "diaper 1"). Fig. 1 is a plan view of the diaper 1 in a developed and stretched state. Fig. 2 is a schematic perspective view when the diaper 1 in a pull-on state is viewed from a front. Fig. 3A is a schematic cross-sectional view taken along a line A-A in Fig. 1. Fig. 3B is a schematic cross-sectional view taken along a line B-B in Fig. 1. The "stretched state" in Fig. 1 is a state where a product (diaper 1) is stretched without crease, specifically, a state where the diaper 1 is stretched until when a dimension of each of the members (for example, a back sheet 30, which is described later) that constitute the diaper 1 becomes a length matching a dimension of the single member or becomes a length close to it.

This diaper 1 has a vertical direction, a lateral direction, and a front-rear direction as three directions that are mutually orthogonal in the pull-on state in Fig. 2. Then, in the following, one side and the other side in the vertical direction in this pull-on state are also referred to as a "waist opening side" and a "crotch side" respectively, and a front side and a rear side in the front-rear direction are also referred to as a "abdominal side" and a "back side" respectively.

On the other hand, in the developed state in Fig. 1, the diaper 1 has a longitudinal direction and a width direction as two directions that are mutually orthogonal. Then, in the following, one side and the other side in the longitudinal direction in this developed state are also referred to as the "abdominal side" and the "back side" respectively. The above-described width direction in the developed state is a direction identical to the above-described lateral direction in the pull-on state. Thus, in the following, the width direction is also referred to as the "lateral direction. " The longitudinal direction in the developed state is a direction along the vertical direction in the pull-on state. As illustrated in Figs. 3A and 3B, a direction perpendicular to the vertical direction (the longitudinal direction) and the lateral direction (the width direction) is defined as a thickness direction. A side that comes into contact with a skin of the target wearer is defined as a "skin side," and its opposite side is defined as a "non-skin side."

As illustrated in Fig. 1, the diaper 1 includes a front waist section 3, a back waist section 4, and a crotch section 5 in the longitudinal direction. The front waist section 3 is a part positioned at the front of the wearer in wearing the diaper 1. The back waist section 4 is a part positioned at the back of the wearer in wearing the diaper 1. Then, the crotch section 5 is provided between the front waist section 3 and the back waist section 4.

The diaper 1 in this embodiment includes an absorbent main body 10, a top sheet 20, a back film 25, the back sheet 30, and leakproof walls 50. Then, from the developed state in Fig. 1, the absorbent main body 10 is folded in two taking a certain position CL10 in the longitudinal direction (the vertical direction) of this absorbent main body 10 as a folding position, and the front waist section 3 and the back waist section 4 that are opposed to one another in this two-folded state are joined using joining means such as welding or adhesion at a front side edge section 3es and a back side edge section 4es. Thus, these waist sections 3, 4 are coupled one another in a ring shape to form the diaper 1 in the pull-on state where a waist opening BH and a pair of leg openings LH, LH are formed, as illustrated in Fig. 2.

The absorbent main body 10 has a function that absorbs excrement such as urine, has an approximately rectangular shape in plan view as illustrated in Fig. 1, and is disposed at a center in the lateral direction while allowing its longitudinal direction to be along the vertical direction of the diaper 1. Then, at one end part (abdominal side) of the absorbent main body 10 in the longitudinal direction, the front waist section 3 constituted of the back sheet 30 and the like is positioned. At the other end part (back side) in the longitudinal direction of the absorbent main body 10, the back waist section 4 constituted of the back sheet 30 and the like is positioned. At both sides of the absorbent main body 10 in the lateral direction, leakproof walls 50 are provided. The absorbent main body 10 includes a liquid-absorbent absorbent core 11, and a core-wrapping sheet 12 that covers an outer peripheral surface of this core 11 (see Fig. 3A).

The absorbent core 11 is formed by shaping liquid absorbent fiber such as pulp fiber into a certain shape, and superabsorbent polymer (what is called a SAP) and the like are mixed inside the absorbent core 11. A length in the lateral direction of the absorbent core 11 (that is, a width of the absorbent core 11) is partially narrowed in the crotch section 5 compared with those at the front waist section 3 and the back waist section 4. At the crotch section 5, depressed sections 11c depressed inside in the lateral direction are formed. The core-wrapping sheet 12 is a liquid-permeable sheet member that covers the outer peripheral surface of the absorbent core 11, and a tissue, a nonwoven fabric or the like can be employed for the sheet 12.

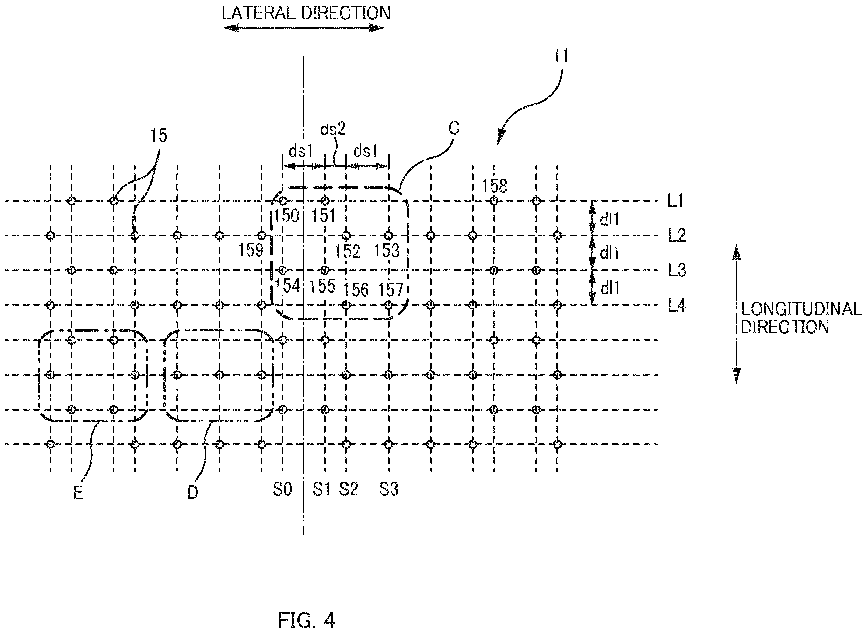

The absorbent core 11 has a surface on which a plurality of dotted compressed portions 15 is disposed (not illustrated in Fig. 1 and Figs. 3A and 3B). Fig. 4 is an explanatory view of disposition of the dotted compressed portions 15. The respective dotted compressed portions 15 are formed using known compressing means such as an embossing process with respect to the thickness direction of the absorbent core 11. At a part where the dotted compressed portions 15 are formed, a density of the absorbent core 11 is high and a thickness of the absorbent core 11 is thin, compared with a part where the dotted compressed portions 15 are not formed.

The plurality of dotted compressed portions 15 are disposed so as to be bilaterally symmetric from a center position in the lateral direction, while having certain intervals in the longitudinal direction (the vertical direction) and the lateral direction of the absorbent core 11. Specifically, in a region C enclosed by the dashed line in Fig. 4, eight dotted compressed portions 150 to 157 are disposed on straight lines L1 to L4 along the lateral direction and straight lines S0 to S3 along the longitudinal direction. The straight lines L1 to L4 are aligned having equal intervals dl1 in the longitudinal direction. The straight lines S0 to S3 are aligned having two kinds of large and small intervals ds1, ds2 (ds1 > ds2) in the lateral direction. In this embodiment, it is approximately defined that the interval dl1 = 7.0 mm, the interval ds1 = 8.5 mm, and the interval ds2 = 4.25 mm.

The interval between the two dotted compressed portions 15 that are adjacent to one another in the longitudinal direction is 2 × dl1. For example, the interval in the longitudinal direction between the dotted compressed portions 151 and 155 disposed on the straight line S1, and the interval in the longitudinal direction between the dotted compressed portions 152 and 156 disposed on the straight line S2 are each 2 × dl1. That is, the dotted compressed portions 15 are disposed at an equal pitch in the longitudinal direction. On the other hand, the interval between the two dotted compressed portions 15 that are adjacent to one another in the lateral direction is not uniform. For example, while the interval in the lateral direction between the dotted compressed portions 150 and 151 disposed on the straight line L1 is ds1 (defined as a first pitch), the interval in the lateral direction between the dotted compressed portion 151 and a dotted compressed portion 158 disposed on its right is 3 × ds1 + 2 × ds2 (defined as a second pitch) . While the interval in the lateral direction between the dotted compressed portions 152 and 153 disposed on the straight line L2 is ds1 (the first pitch), the interval in the lateral direction between the dotted compressed portion 152 and a dotted compressed portion 159 disposed on its left side is ds1 + 2 × ds2 (defined as a third pitch) . That is, the dotted compressed portions 15 are disposed at a plurality of kinds of pitches (the above-described first to third pitches) in the lateral direction. In other words, the dotted compressed portions 15 have parts disposed at non-uniformpitches in the lateral direction.

The diaper 1 has a pull-on shape as illustrated in Fig. 2 in wearing. At this time, the planar absorbent core 11 (absorbent main body 10) is appropriately deformed in conformity to the body shape of the wearer, and thus the fitting performance of the diaper 1 can be improved, and the leakage of the excrement can be likely to be restrained. The absorbent core 11 in this embodiment includes the plurality of dotted compressed portions 15. Accordingly, the respective dotted compressed portions 15 become base points for folding to facilitate natural deformation of the absorbent core 11 in wearing the diaper 1.

First, since the respective dotted compressed portions 15 are provided at the equal pitch in the longitudinal direction, the base points for folding are likely to be equally formed in the longitudinal direction at the absorbent core 11. That is, the absorbent core 11 is likely to uniformly deform over the whole in longitudinal direction. In wearing the diaper 1, it is necessary for the absorbent core 11 to be roundly deformed in the longitudinal direction from the front to the back of the wearer via a crotch portion. Accordingly, as in this embodiment, by allowing the absorbent core 11 to be likely to uniformly deform in the longitudinal direction, the absorbent core 11 is more likely to effortlessly fit along the roundness of the body.

Next, since the respective dotted compressed portions 15 are disposed at the unequal pitch in the lateral direction, a region where the base points for folding are likely to be formed and a region where the base points for folding are less likely to be formed exist in the lateral direction at the absorbent core 11. In wearing the diaper 1, the absorbent core 11 includes parts which need to be deformed in the lateral direction, for example, a part that comes into contact with the crotch of the wearer needs to be formed into a planar shape, and a part from the crotch to the groin needs to be formed into a curved shape. Accordingly, as in the present embodiment, by mixing the part where the pitch of the dotted compressed portions 15 is narrow and the base points for folding are densely formed and the part where the pitch of the dotted compressed portions 15 is wide and the base points for folding are sparsely formed in the lateral direction, the absorbent core 11 can be concentratedly deformed and can be made to be less likely to deform from the planar shape. That is, this can make the absorbent core 11 be likely to freely deform in the lateral direction while maintaining the plane according to the body shape of the wearer. This makes the absorbent core 11 be likely to fit the body.

In the body of the wearer, while the crotch portion constitutes a complicated curved surface, the vicinity of the waist portion has a shape relatively close to a plane. In contrast, at the absorbent core 11 in this embodiment, a part whose disposition density of the dotted compressed portions 15 is high and a part whose disposition density of the dotted compressed portions 15 is low exist. In other words, the absorbent core 11 has a part where the number of dotted compressed portions disposed per unit area is large and a part where the number of dotted compressed portions disposed per unit area is small. For example, regions D and E enclosed by the two-dot chain lines in Fig. 4 are regions both having an identical area. However, while three dotted compressed portions 15 are disposed in the region D, six dotted compressed portions 15 are disposed in the region E. The density of the dotted compressed portions 15 at the absorbent core 11 in the region E is higher than that in the region D. That is, the base points for folding are likely to be formed in the region E compared with in the region D. Thus, the region E is likely to deform into the curved shape. Accordingly, having the region that is likely to deform (for example, the region E) and the region that is less likely to deform (for example, the region D) on the identical plane restrains the absorbent core 11 from being likely to deform only in a specific direction (from getting deformation habit) . That is, the deformation of the absorbent core 11 becomes irregular to be likely to follow the complicated curved surface of the body. In wearing the diaper 1, this allows the surface fitting of the absorbent core 11 with respect to the body of the wearer to be likely achieved. Moreover, follow ability of the absorbent core 11 with respect to body movements of the wearer increases.

The top sheet 20 is a liquid-permeable sheet member that is disposed at the skin side of the absorbent main body 10 in the thickness direction and comes into contact with the skin of the wearer in wearing the diaper 1. The top sheet 20 in this embodiment is formed by, for example, an air-through nonwoven fabric or a spunbond nonwoven fabric.

The back film 25 is a liquid-impermeable and moisture-permeable sheet member disposed at the non-skin side of the absorbent main body 10, and formed by, for example, a resin film. Providing the back film 25 restrains water content such as urine absorbed by the absorbent main body 10 from moving to (penetrating) a clothes side (the non-skin side) of the wearer. On the non-skin side of the back film 25, an inner back sheet (not illustrated in Figs. 3A and 3B) such as the nonwoven fabric may be layered.

The back sheet 30 is a sheet member disposed on the non-skin side in the thickness direction with respect to the absorbent main body 10 to constitute an outer layer of the diaper 1, and also a member that constitutes the front waist section 3 and the back waist section 4. In this embodiment, the back sheet 30 includes an upper outer-layer sheet 32 and a lower outer-layer sheet 33 (see Figs. 3A and 3B). The upper outer-layer sheet 32 and the lower outer-layer sheet 33 are flexible sheet-shaped members stuck in the thickness direction to be disposed on the non-skin side with respect to the back film 25 and non-skin side end parts of the leakproof walls 50, and formed by, for example, the spunbond nonwoven fabric. The upper outer-layer sheet 32 and the lower outer-layer sheet 33 both have identical shapes at at least the front waist section 3 and the back waist section 4, and have approximately hourglass shapes as illustrated in Fig. 1 as a whole.

Elastic members such as elastic strings are provided between the upper outer-layer sheet 32 and the lower outer-layer sheet 33 in the thickness direction. In this embodiment, as illustrated in Fig. 1, a plurality of front waist elastic members 35a are sandwiched between the upper outer-layer sheet 32 and the lower outer-layer sheet 33 to be joined in a stretched state in the lateral direction at a certain stretch scaling, at the front waist section 3 (a region between the front side edge sections 3es, 3es) of the diaper 1. These front waist elastic members 35a apply stretchability in the lateral direction to the front waist section 3 of the diaper 1.

The "stretch scaling" of the elastic member indicates a stretch degree when a natural length of the elastic member (the elastic string) is 1. For example, when the stretch scaling is 1.2, the elastic member has been stretched by 0.2 from the natural length.

In the front waist section 3, in a lower region in the vertical direction than a region on which the front waist elastic members 35a are disposed and at a lower end part in the vertical direction (that is, a part along the leg opening LH when the diaper 1 is form into the pull-on shape), a front waist lower-end-part elastic member 36lg (hereinafter also simply referred to as an "elastic member 36lg") is sandwiched between the upper outer-layer sheet 32 and the lower outer-layer sheet 33 to be joined in a state stretched in the lateral direction at a certain stretch scaling. This elastic member 36lg applies stretchability along the leg opening LH to the lower end part of the front waist section 3. This elastic member 36lg has straight sections 36lgs disposed along the lateral direction at both sides in the lateral direction, and an inclined section 36lgc inclined obliquely upward from the outside toward the inside in the lateral direction between the straight sections 36lgs. The inclined section 36lgc is disposed at least partially overlapping the absorbent main body 10 in the thickness direction. Then, at least a part of the inclined section 36lgc is discontinuous at a center part in the lateral direction. Accordingly, in a region that is a discontinuous section, the stretchability of the elastic member 36lg does not act on the front waist section 3 and the absorbent main body 10. The function of the elastic member 36lg and its action will be described later.

On the other hand, at the back waist section 4 (a region between the back side edge sections 4es, 4es) of the diaper 1, between the upper outer-layer sheet 32 and the lower outer-layer sheet 33 in the thickness direction, a plurality of back waist elastic members 35b are joined between the upper outer-layer sheet 32 and the lower outer-layer sheet 33 in a state stretched in the lateral direction at a certain stretch scaling. These back waist elastic members 35b apply stretchability in the lateral direction to the back waist section 4 of the diaper 1. At a lower end part of the back waist section 4 in the vertical direction (a part along the leg opening LH when the diaper 1 is formed into the pull-on shape), a back waist lower-end-part elastic member 37lg (hereinafter also simply referred to as an "elastic member 37lg") is sandwiched between the upper outer-layer sheet 32 and the lower outer-layer sheet 33 to be joined in a state stretched at a certain stretch scaling. This elastic member 37lg applies stretchability along the leg opening LH to the lower end part of the back waist section 4.

The leakproof walls 50 correspond to barrier cuff sections that stand to the skin side of the wearer from both end parts of the absorbent main body 10 in the lateral direction to restrain the excrement such as urine excreted into the absorbent main body 10 from leaking outside the diaper 1. A leakproof-wall elastic member 55 such as an elastic string is joined to the leakproof wall 50 in a state stretched at a certain stretch scaling along the longitudinal direction. In wearing the diaper 1, the stretchability of this leakproof-wall elastic member 55 acts to allow the leakproof wall 50 to stand to the skin side, so that the leakproof wall 50 fits along the leg (the groin) of the wearer.

### Fitting Performance of Diaper 1

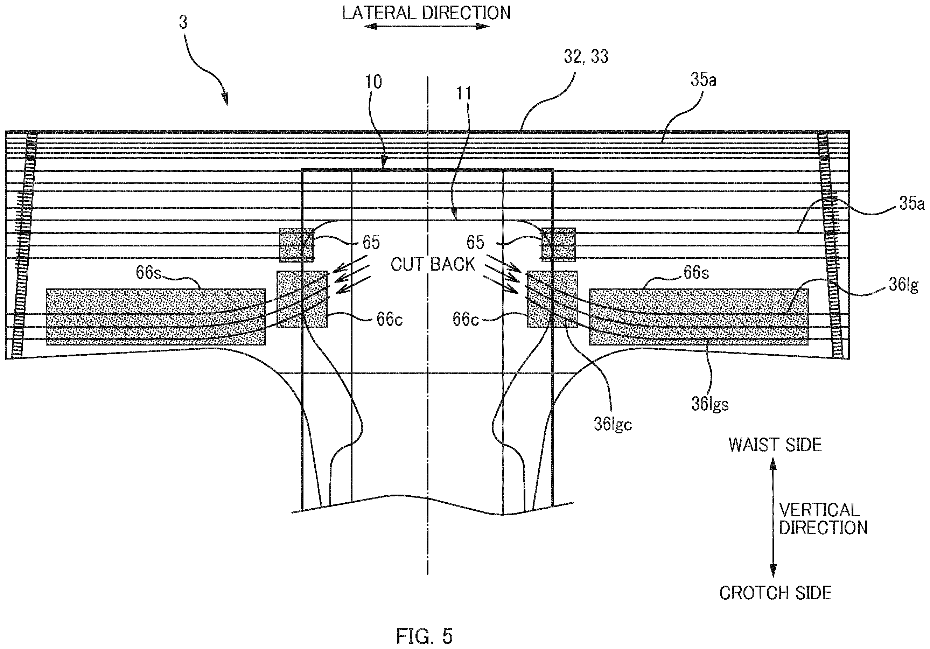

The following describes action by the front waist lower-end-part elastic member 361g (the elastic member 361g) and the front waist elastic member 35a provided on the front waist section 3. Fig. 5 is an enlarged plan view illustrating the front waist section 3 in the developed and stretched state. As described above, the elastic member 36lg has the straight sections 36lgs and the inclined section 36lgc, and at least a part of the elastic member 36lg is discontinuous at the center part in the lateral direction.

When the discontinuous section is formed on the elastic member 36lg in a manufacturing process of the diaper 1, first, an adhesive such as a hot melt adhesive is applied on a certain region between the upper outer-layer sheet 32 and the lower outer-layer sheet 33 of the back sheet 30 in the thickness direction in the front waist section 3, thus forming an adhesion region 66 (an elastic-member adhesion region 66). Then, the elastic member 36lg is disposed so as to overlap this adhesion region in a state stretched in the lateral direction and an oblique direction, and thus the elastic member 361g is joined to the front waist section 3. Fig. 5 illustrates straight-section adhesion regions 66s for mainly joining the straight sections 36lgs, and inclined-section adhesion regions 66c for mainly joining the inclined section 36lgc by hatching.

Next, the elastic member 36lg is cut at a certain position at the center part in the lateral direction. In other words, the elastic member 36lg is cut in a region between right and left pair of inclined-section adhesion regions 66c, 66c in the lateral direction. Then, as illustrated by arrows in Fig. 5, the elastic member 36lg starts contracting from a cut portion (cut ends) toward the inclined-section adhesion regions 66c at both sides in the lateral direction, and stops contracting at the inclined-section adhesion regions 66c. This operation is also referred to as "cut back" of the elastic member. In this embodiment, in the region between the inclined-section adhesion regions 66c, 66c in the lateral direction, the elastic member 36lg is not joined (or is joined with weak adhesion force to the extent that the stretching force does not act). Accordingly, when the elastic member 361g is cut back, the upper outer-layer sheet 32 and the lower outer-layer sheet 33 that constitute the front waist section 3 do not contract, and only the elastic member 36lg contracts to an oblique lower side along an inclined direction of the inclined-section adhesion region 66c. In other words, the elastic member 36lg is displaced relative to the front waist section 3. This forms the discontinuous section of the elastic member 36lg at the center part of the diaper 1 in the lateral direction.

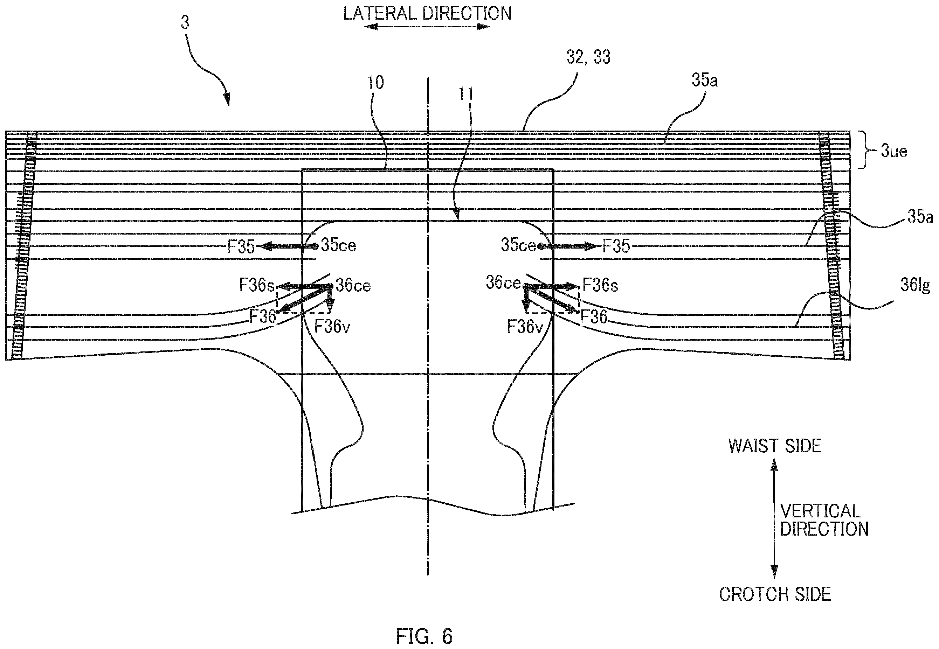

Fig. 6 is an explanatory view of action of force by the elastic member 36lg in wearing the diaper 1. In wearing the diaper 1, when operation that opens the waist opening BH is performed, the front waist section 3 is expanded in the lateral direction so that the elastic member 36lg stretches in the lateral direction to develop contractile force around the leg of the lower end part of the front waist section 3 in the vertical direction. Due to this contractile force, the front waist section 3 fits to the body of the wearer. Accordingly, in the elastic member 36lg, outer end parts in the lateral direction of the straight sections 36lgs and the inclined section 36lgc are in a state that fits (are fixed) along the waist (the leg) of the wearer. On the other hand, a lateral-direction inner end part 36ce of the inclined section 36lgc is a free end side that has been cut to cut back, and is not fixed to the waist of the wearer. Accordingly, by the action of the contractile force of the elastic member 36lg, a force F36 from the inside to the outside in the lateral direction and from the upper side toward the lower side in the vertical direction acts on the lateral-direction inner end part 36ce of the inclined section 36lgc. Then, the force F can be divided into a force F36s toward the outside in the lateral direction and a force F36v toward the lower side in the vertical direction as illustrated in Fig. 6.

In wearing the diaper 1, by the force F36s toward the outside in the lateral direction, a region overlapping the lateral-direction inner end part 36ce in the thickness direction in the front waist section 3 and the absorbent main body 10 (the absorbent core 11 is pulled outside in the lateral direction). This can widen the absorbent main body 10 (the absorbent core 11) in the lateral direction together with the front waist section 3, thus making the excrement such as urine be less likely to leak from the absorbent main body 10 at the front of the diaper 1.

By the force F36v toward the lower side in the vertical direction, a region overlapping the lateral-direction inner end part 36ce in the thickness direction in the front waist section 3 and the absorbent main body 10 (the absorbent core 11) is pulled to the lower side. Here, in the front waist section 3, the front waist elastic members 35a are continuously disposed (see Fig. 5) in the lateral direction at an upper end part 3ue in the vertical direction (an upper region with respect to an upper end of the absorbent main body 10). Thus, by the stretching force of these elastic members 35a, the upper end part 3ue is fixedly fitted to the waist of the wearer. On the other hand, the lower end part of the front waist section 3 in the vertical direction is pulled to the lower side by the force F36v. Accordingly, the lower end part of the front waist elastic member 35a is pulled to the lower side in a state where the upper end part 3ue is fixed. This makes the front waist section 3 be likely to be widened in the vertical direction.

In a conventional disposable diaper, there is a problem that when the wearer moves his/her own body such as taking the forward-inclined posture, a waist belt section corresponding to the front waist section 3 is likely to bend in the vertical direction and generate riding-up. In contrast, in the diaper 1 in this embodiment, the force that widens the front waist section 3 in the vertical direction acts in wearing. Thus, the bending and the riding-up are less likely to occur to ensure improvement of the fitting performance of the front waist section 3. Then, the front waist section 3 fixedly fits the body of the wearer in the widened state, so that the absorbent main body 10 can be more strongly supported. For example, even when the absorbent main body 10 absorbs the excrement and increases its weight, the front waist section 3 is less likely to be displaced off from the body of the wearer. This can act against drooping-down of the absorbent main body 10 thereby enabling the leakage or the like of the excrement to be easily restricted.

As described above, the absorbent core 11 in this embodiment is likely to deform corresponding to the body shape of the wearer since the base points for folding is likely to be formed by the plurality of dotted compressed portions 15. On the other hand, when the wearer moves his/her own body, many creases are likely to be formed on the surface of the absorbent core 11 and the absorbent core 11 is likely to bend, thus possibly causing the leakage of the excrement. However, the force F36v and the force F36s also act on the absorbent core 11 to widen the absorbent core 11. Thus, the absorbent core 11 becomes likely to fit the body of the wearer. This can efficiently restrain the leakage or the like of the excrement.

The following describes the front waist elastic member 35a. In the front waist elastic member 35a, at least a part of the center part in the lateral direction is discontinuous in a region overlapping the absorbent main body 10 in the longitudinal direction . As in the above-described case of the elastic member 361g, this discontinuous section is formed such that the front waist elastic member 35a is cut at the center part in the lateral direction to be cut back. Specifically, similarly to the above-described adhesion region 66, the front waist elastic member 35a is cut between a pair of adhesion regions 65, 65 (waist elastic-member adhesion regions 65) formed at right and left in the lateral direction. This allows the front waist elastic member 35a to contract from cut ends toward the adhesion regions 65 at both sides in the lateral direction (see Fig. 5).

Since a part of the front waist elastic member 35a is discontinuous at the center part in the lateral direction, similarly to the case of the elastic member 36lg, a force F35 from the inside toward the outside in the lateral direction acts on a lateral-direction inner end part 35ce (that is, a part including the cut end) of the front waist elastic member 35a (see Fig. 6). Accordingly, in the front waist section 3 and the absorbent main body 10, regions overlapping the lateral-direction inner end parts 35ce in the thickness direction are pulled outward in the lateral direction. This facilitates the absorbent main body 10 widening in the lateral direction to allow the excrement such as urine to be less likely to leak from the absorbent main body 10 at the front of the diaper 1.

A position of the lateral-direction inner end part 35ce of the front waist elastic member 35a and a position of the lateral-direction inner end part 36ce of the front waist lower-end-part elastic member 36lg (the elastic member 36lg) are different in the lateral direction. Thus, the range on which the contractile force by the front waist elastic member 35a acts differs from the range on which the contractile force by the elastic member 36lg acts in the lateral direction. In the example in Fig. 5 and Fig. 6, the lateral-direction inner end part 36ce of the elastic member 36lg is positioned on the inner side in the lateral direction with respect to the lateral-direction inner end part 35ce of the front waist elastic member 35a. This is because the range in which the adhesion region 66 (the inclined-section adhesion region 66c) that stops the cut back of the elastic member 36lg is formed differs from the range in which the adhesion region 65 that stops the cut back of the front waist elastic member 35a is formed.

In this way, differentiating the forming ranges of the adhesion regions 65, 66 can differentiate the range on which the force F35 by the front waist elastic member 35a acts from the range on which the force F36 by the elastic member 36lg acts. Especially, the force F36 that acts due to the elastic member 36lg has a constituent (F36v) directed downward in a downward direction. Thus, by adjusting the range in which the adhesion region 66 is formed, the range on which the force F36 acts can be adjusted. This can change the point of action and the magnitude of force when the front waist section 3 is pulled downward, thereby enabling the fitting feeling of the target wearer to be improved. For example, it becomes possible to adjust or the like widening strength of the front waist section 3 in the vertical direction, depending on the age and the size of the body of the target wearer.

The lateral-direction inner end part 35ce of the front waist elastic member 35a overlaps the absorbent main body 10 in the lateral direction; however, does not necessarily overlap the absorbent core 11. When the elastic member 35a does not overlap the absorbent core 11, the force F35 generated by the stretchability of the front waist elastic member 35a acts on both end parts of the absorbent main body 10 in the lateral direction, but is less likely to act on the absorbent core 11. Since the absorbent core 11 has a high rigidity, if the front waist elastic member 35a overlapped the absorbent core 11, the front waist elastic member 35a would be less likely to contract in the lateral direction in some cases. In this case, since a contraction amount of the front waist section 3 in the lateral direction decreases, the fitting performance of the front waist section 3 in wearing the diaper 1 possibly deteriorates. In contrast, if the front waist elastic member 35a is allowed not to overlap the absorbent core 11, the contraction becomes likely to be performed over the wide range of the front waist section 3 in the lateral direction to improve the fitting performance of the front waist section 3 in wearing the diaper 1.

In the example in Fig. 5, the adhesion region 65 and the adhesion region 66 (the inclined-section adhesion region 66c) are formed at positions displaced off one another. That is, the adhesion region 65 and the adhesion region 66 are not overlapped in the thickness direction. This allows the force F35 due to the contractile force of the front waist elastic member 35a and the force F36 due to the contractile force of the front waist lower-end-part elastic member 36lg (the elastic member 36lg) to act independently one another. In other words, the force F35 due to the front waist elastic member 35a and the force F36 due to the elastic member 36lg become less likely to interfere with one another. If the respective forces are interfered, and, for example, the contractile force in the lateral direction due to the front waist elastic member 35a acts on the elastic member 36lg, the direction where the absorbent main body 10 and the front waist section 3 are pulled may be changed. Accordingly, the assumed widening effect possibly cannot be obtained, and the fastening by the respective elastic members 35a, 36lg may become locally strong. In contrast, by allowing the force F35 and the force F36 to be less likely to interfere with one another as in the present embodiment, the force F35 and the force F36 are each allowed to precisely and easily act on the front waist section 3 as described above, thus further improving the fitting performance of the front waist section 3.

### Modification

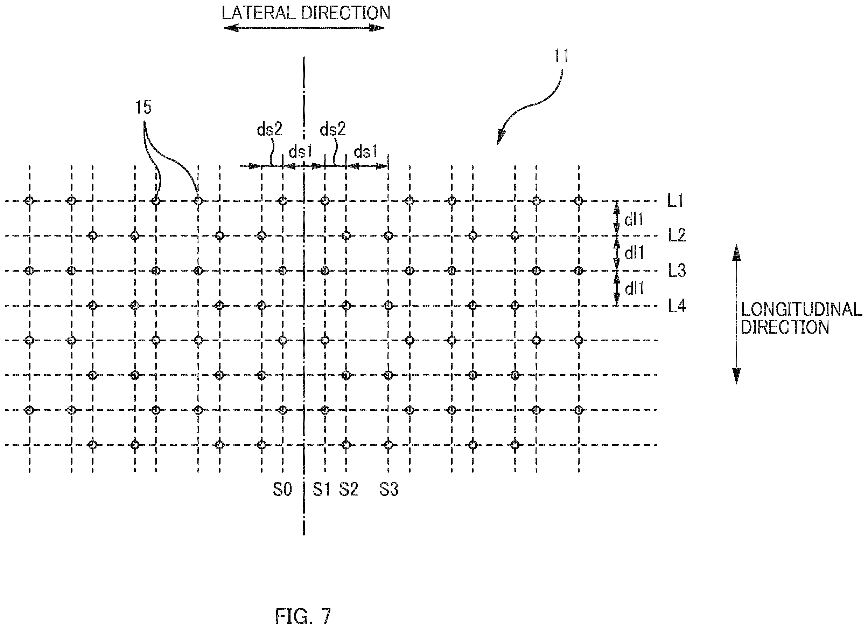

In Fig. 4, the disposition of the plurality of dotted compressed portions 15 provided on the absorbent core 11 has been described. However, the disposition of the dotted compressed portions 15 is not limited to the case illustrated in Fig. 4. Fig. 7 is an explanatory view of a modification of the disposition of the dotted compressed portions 15.

In the example of Fig. 7, similarly to the case in Fig. 4, the straight lines L1 to L4 along the lateral direction are aligned with the equal intervals dl1 in the longitudinal direction, and the straight lines S0 to S3 along the longitudinal direction are aligned with two kinds of large and small intervals ds1, ds2 (ds1 > ds2) in the lateral direction. Then, the respective dotted compressed portions 15 are disposed such that the interval between two dotted compressed portions 15, 15 adjacent to one another in the longitudinal direction is 2 × dl1, and the intervals between two dotted compressed portions 15, 15 adjacent to one another in the lateral direction alternately repeat ds1 (the first pitch) and ds1 + 2 × ds2 (the third pitch). Accordingly, similarly to the case in Fig. 4, the dotted compressed portions 15 provided equally in the longitudinal direction facilitate uniform deformation of the absorbent core 11 in the longitudinal direction. The dotted compressed portions 15 provided unequally in the lateral direction can deform the absorbent core 11 concentratedly in the lateral direction, and make the absorbent core 11 less likely to deform from the planar shape.

On the other hand, while the interval in the lateral direction of the dotted compressed portions 15 has three kinds in Fig. 4, the interval in the lateral direction of the dotted compressed portions 15 has two kinds in this modification as illustrated in Fig. 7. Thus, the disposition of the dotted compressed portions 15 is close to equal at the whole surface of the absorbent core 11. That is, the number of the dotted compressed portions disposed per unit area is likely to become equal. Thus, compared with the case in Fig. 4, the absorbent core 11 is likely to maintain its planar shape.

Thus changing the disposition of the dotted compressed portions 15 appropriately allows adjustment of deformability of the absorbent core 11 in wearing. For example, when the target wear is an adult man whose body is large, it is prioritized that the absorbent core 11 is allowed to be likely to maintain the plane to enhance the fitting performance in a wide area. On the other hand, when the target wear is a child whose body is small, it is prioritized that the absorbent core 11 is allowed to be likely to complexly deform to enhance the fitting performance to the curved surface. Thus, the fitting performance of the absorbent core 11 can be adjusted in an appropriate state. Needless to say, the disposition of the dotted compressed portions 15 is not limited to the modification in Fig. 7, and may be another disposition.

### === Other Embodiments ===

Hereinabove, although embodiments of the present invention have been described, the above embodiments are for facilitating understanding of the present invention and are not for interpreting the present invention in a limited way. And it is needless to say that modifications and improvements of the present invention are possible without departing from the scope of the invention, and equivalents thereof are also encompassed by the invention. For example, the following modifications are possible.

In the above-described embodiment, the absorbent main body 10 that includes the absorbent core 11 including the plurality of dotted compressed portions 15 has been described. However, the configuration of the absorbent main body 10 is not limited thereto. For example, the absorbent main body 10 may have a configuration where the absorbent core 11 does not have the dotted compressed portions 15, or a configuration where low weight portions or low density portions (for example, linear grooves) different from the dotted compressed portions 15 are formed on the surface of the absorbent core 11. The absorbent main body 10 may have a multi-layer structure where the absorbent core 11 is layered in the thickness direction. Even in these cases, the above-described effect by the inclined section 361gc can improve the fitting performance of the front waist section 3.

In the above-described embodiment, the elastic members 361g are symmetrically disposed with respect to the center part of the diaper 1 in the lateral direction (see Fig. 1). However, the elastic members 361g may be asymmetrically disposed. For example, the inclination angles of the inclined section 361gc with respect to the lateral direction or the lengths of the inclined section 361gc may be different at right and left. Such asymmetric elastic members 361g allow adjustment of the magnitude and the direction of the force that acts on the front waist section 3. This facilitates the achievement of the appropriate fitting performance in wearing the diaper 1, corresponding to the condition of the target wearer, for example, when the target wearer is a person whose one hand is paralyzed, by putting emphasis on facilitating the operation that raises the diaper 1 (the front waist section 3) at one side in the lateral direction, and putting emphasis on the improvement of the fitting performance of the front waist section 3 at the other side in the lateral direction.

In the elastic member 36lg, coupling portions of the inclined section 36lgc to the straight sections 36lgs may be curved at a certain curvature. In other words, the elastic member 36lg may include curving sections 36w curved between the inclined section 36lgc and the straight sections 36lgs (see Fig. 1). The inclined section 36lgc and the straight sections 36lgs are smoothly coupled by such curving sections 36w. This can cause the stretching force by the straight sections 36lgs and the stretching force by the inclined section 36lgc to smoothly coordinate, thus facilitating fitting of the front waist section 3 to the body of the wearer as a whole. At this time, the curvatures of the curving sections 36w may be different at right and left with respect to the center of the diaper 1 in the lateral direction. Similarly to the above-described case where the inclination angles of the inclined section 36lgc are each different, asymmetrically disposing the elastic members 36lg facilitates the achievement of the optimum fitting performance in wearing the diaper 1 according to the condition of the target wearer (for example, the person whose one hand is paralyzed).

In the above-described embodiment, the example where the elastic strings are used as the waist elastic member and the leakproof-wall elastic member has been described. However, these elastic members are not limited to the linear elastic members such as so-called elastic strings. For example, planar (strip-shaped) elastic members having a certain width may be used. By using the sheet members having stretch (for example, stretch nonwoven fabrics) as the sheet members that constitute the leakproof walls and the back sheet, a configuration where there is no need to additionally dispose the elastic members such as the elastic strings may be employed.

## Claims

1. A pull-on disposable diaper having a vertical direction, a lateral direction and a thickness direction that are orthogonal to one another, the pull-on disposable diaper comprising:
an absorbent main body that absorbs liquid;
a front waist section positioned at one end part of the absorbent main body in a longitudinal direction; and
a back waist section positioned at another end part of the absorbent main body in the longitudinal direction,
the front waist section having a lower end part on which an elastic member that is extendable in the lateral direction is provided,
the elastic member including an inclined section that is inclined obliquely upward from an outside toward an inside in the lateral direction, at least in a region where the elastic member overlaps with the absorbent main body in the thickness direction,
the elastic member being discontinuous at a center part of the absorbent main body in the lateral direction.

2. The pull-on disposable diaper according to claim 1, wherein
the absorbent main body includes a liquid-absorbent absorbent core, and
the elastic member includes a part overlapping with the absorbent core in the thickness direction.

3. The pull-on disposable diaper according to claim 2, wherein
the absorbent core has a surface on which a plurality of dotted compressed portions are provided, and
the plurality of dotted compressed portions are disposed at an equal pitch in the vertical direction.

4. The pull-on disposable diaper according claim 2 or 3, wherein
the absorbent core has a surface on which a plurality of dotted compressed portions are provided, and
the plurality of dotted compressed portions are disposed with a first pitch and a second pitch different in length from the first pitch in the lateral direction.

5. The pull-on disposable diaper according to any one of claims 2 to 4, wherein
the absorbent core has a surface on which a plurality of dotted compressed portions are provided, and
the absorbent core includes a part where a number of the dotted compressed portions disposed per unit area is large, and a part where a number of the dotted compressed portions disposed per unit area is small.

6. The pull-on disposable diaper according to any one of claims 1 to 5, wherein
the front waist section includes a waist elastic member that is extendable in the lateral direction, in an upper region in the vertical direction with respect to a region where the elastic member is disposed,
at least a part of the waist elastic member is discontinuous at a center part in the lateral direction, and
a position of an inner end of the elastic member and a position of an inner end of the waist elastic member are different in the lateral direction.

7. The pull-on disposable diaper according to claim 6, wherein
the front waist section includes:
an inclined-section adhesion region to which the inclined section is joined; and
a waist-elastic-member adhesion region to which the waist elastic member is joined without overlapping the inclined-section adhesion region.

8. The pull-on disposable diaper according to claim 6 or 7, wherein
the absorbent main body includes a liquid-absorbent absorbent core, and
the waist elastic member does not have a part overlapping with the absorbent core in the thickness direction.

9. The pull-on disposable diaper according to any one of claims 1 to 8, wherein
a right and left pair of the elastic members are provided along the lateral direction, and
the inclined section has inclination angles with respect to the lateral direction, which are different at right and left.

10. The pull-on disposable diaper according to any one of claims 1 to 9, wherein
a right and left pair of the elastic members are provided along the lateral direction,
the elastic members each include a straight section disposed along the lateral direction, and a curving section curved at a certain curvature between the inclined section and the straight section, and
curvatures of the curving sections are different at right and left.
